# EUROPEAN PATENT SPECIFICATION

(11) **EP 4 424 399 B1**
(45) Date of publication and mention of the grant of the patent: **29.04.2026**
(21) Application number: 23892744.6
(22) Date of filing: 25.05.2023
(51) Int. Cl.: B01D 9/02, B01D 36/00, C07C 29/74, C07C 29/76, C07C 29/78, C07C 31/18

(54) **SYSTEM AND METHOD FOR PREPARING HIGH-QUALITY XYLITOL CRYSTALS**
SYSTEM UND VERFAHREN ZUR HERSTELLUNG VON XYLITOLKRISTALLEN HOHER QUALITÄT
SYSTÈME ET PROCÉDÉ DE PRÉPARATION DE CRISTAUX DE XYLITOL DE HAUTE QUALITÉ

(30) Priority: 09.12.2022 CN 202211583786
(43) Date of publication of application: 04.09.2024
(73) Proprietor: Zhejiang Huakang Pharmaceutical Co., Ltd., Quzhou, Zhejiang 324302 (CN)
(72) Inventor: WU, Qiang, Quzhou, Zhejiang 324302 (CN); LI, Mian, Santa Clara CA (US); ZHANG, Wenyao, Quzhou, Zhejiang 324302 (CN); YANG, Wulong, Quzhou, Zhejiang 324302 (CN); XU, Weidong, Quzhou, Zhejiang 324302 (CN); QIN, Shufang, Quzhou, Zhejiang 324302 (CN); ZHEN, Ni, Quzhou, Zhejiang 324302 (CN)
(74) Representative: Sun, Yiming
(86) International application number: PCT/CN2023/096366
(87) International publication number: WO 2024/119733

(56) References cited:
- CN-A- 108 837 550
- CN-A- 111 777 493
- CN-A- 111 777 493
- CN-A- 112 442 556
- CN-A- 112 661 796
- CN-A- 115 784 843
- CN-A- 115 888 165
- CN-U- 218 910 200
- CN-U- 218 910 201
- CN-U- 218 910 201
- US-A- 4 066 711

## Description

### TECHNICAL FIELD

The present disclosure relates to the technical field of xylitol preparation, and in particular to a preparation system and a preparation method for high-quality xylitol crystals.

### BACKGROUND

At present, the industrial production of xylitol is primarily based on the chemical hydrogenation method, specifically, agricultural fiber waste containing hemicellulose is hydrolyzed with dilute acid, then a hydrolysate is neutralized, decolorized, concentrated, and crystallized to obtain xylose crystals, the xylose crystals are then dissolved into a specific concentration of xylose solution, which is reduced to xylitol by hydrogenation, and finally xylitol crystals are obtained by performing ion exchange, vacuum concentration, reduced temperature crystallization, centrifugal drying, and other steps on the xylitol. In order to improve the yield of xylitol products, a centrifugal mother liquor is often blended with a hydrogenation solution, resulting in the presence of 4-6% heterosaccharides and sugar alcohols in a blending solution. These heterosaccharides and sugar alcohols have a low influence on a crystallization process of xylitol, but reduce the pH of the product, which ultimately leads to a great difference in the flavor of the product.

In the production process of xylitol, two main factors affect the pH of the product, one of which is an ion exchange process. When a xylitol material solution passes through cation and anion exchange columns, H⁺ and OH⁻ in a resin are exchanged out, respectively. Due to a difference in an exchange amount of the cation and anion exchange columns, finally prepared xylitol crystals may have different pH. The other factor affecting the pH of the product is a difference in a composition of a xylitol concentrate, which leads to a great difference in the pH of the xylitol crystal. Since acidity coefficients (pKa) of heterosaccharides such as glucose and xylose are smaller than an acidity coefficient of xylitol, as shown in Table 1, when there is a certain amount of heterosaccharides in the xylitol concentrate, the prepared xylitol crystals have a relatively low pH and relatively variable flavor, as shown in Table 2.

**Table 1 Table of acidity coefficients of common sugar alcohols**

| Category | Xylitol | Glucose | Xylose | Arabinose | galactose |
|---|---|---|---|---|---|
| Acidity coefficient (pKa) | 13.24±0.2 | 12.43 | 12.14 | 12.46±0.2 | 12.35 |

**Table 2 pH of xylitol crystals prepared from xylitol concentrates with different compositions**

| Composition of xylitol concentrate | 100% xylitol | 97% xylitol + 3% sorbitol | 97% xylitol + 3% glucose |
|---|---|---|---|
| pH of xylitol crystals | 6.02 | 5.72 | 5.76 |

At present, there are mainly two methods to increase the pH of the xylitol crystals, one of which is to add a group of strong alkali resin or mixed bed resin after the ion exchange process, as disclosed in the patent references of CN109438184A and CN110894180A; the other of which is to add a small amount of alkali to an ion exchange solution, as disclosed in the patent reference of CN112661796A. Both methods achieve the purpose of increasing the pH of the xylitol crystals by increasing the pH of the xylitol concentrate. These methods are simple and clear in process route, and have corresponding technical effects. However, the problem that the xylitol prepared by the conventional process has a relatively low pH and a great flavor difference is not essentially solved.

Therefore, in view of the problem in the conventional production process of xylitol, it is important to provide a method for increasing the pH of the xylitol crystals and improving the flavor of the xylitol products from the intrinsic nature of the low pH and the great flavor difference of the xylitol crystals.

### SUMMARY

The technical problem to be solved in the present disclosure is to provide a preparation system and a preparation method for high-quality xylitol crystals. First prepared xylitol crystals are dissolved by adding water in a certain proportion to obtain a xylitol solution with a higher purity, and then the xylitol solution is recrystallized to prepare xylitol crystals with a higher pH and better flavor. The preparation system not only obviously improves the pH of the xylitol crystals and maintains the pH stable for a long time, but also effectively improves the flavor of the xylitol crystals.

The present disclosure is implemented by providing a preparation system and a preparation method for high-quality xylitol crystals. The preparation system comprises a blending tank, a heat exchanger, a decolorization tank, an ion exchange system, a microporous filter, a first evaporator, a first crystallization kettle, a first centrifuge, a fluidization drying bed, a xylitol dissolution tank, a second evaporator, a second crystallization kettle, a second centrifuge, a hot air drying tank, and a cold air fluidization bed which are sequentially connected through pipelines. The first centrifuge and the second centrifuge are provided with a liquid outlet, respectively. The liquid outlet of the first centrifuge is connected with an inlet of the blending tank through a pipeline. The liquid outlet of the second centrifuge is connected with an inlet of the xylitol dissolution tank through a pipeline. The blending tank is provided with an inlet for a material to be treated. The xylitol dissolution tank is provided with a water inlet for purified water. The cold air fluidization bed is provided with an outlet. The material to be treated is a xylitol hydrogenation solution. A material output from the outlet of the cold air fluidization bed is a xylitol crystal product.

The technical problem to be solved by the present disclosure is to further provide a preparation method for high-quality xylitol crystals. The preparation method may comprise the following steps:
step 1, preparing a xylitol concentrate, including blending and mixing a xylitol hydrogenation solution with a primary centrifugal mother liquor delivered from a first centrifuge in a certain proportion to obtain a mixed material solution, and then performing a heat exchange treatment by a heat exchanger, a decolorization treatment by a decolorization tank, an ion exchange treatment by an ion exchange system, a filtration treatment by a microporous filter, and an evaporation treatment by a first evaporator on the mixed material solution, respectively, to obtain the xylitol concentrate; wherein a concentration of the xylitol concentrate is within a range of 1200-1400 g/L, and a purity of the xylitol concentrate is within a range of 94-96%;
step 2, crystallizing the xylitol concentrate, including cooling and crystallizing the xylitol concentrate in a first crystallization kettle for 8-12 h to obtain a xylitol sugar paste, performing a separation treatment by the first centrifuge and a drying treatment by a fluidization drying bed on the xylitol sugar paste to obtain a crude xylitol crystal, and the primary centrifugal mother liquor obtained by the separation treatment of the first centrifuge back-setting to the blending tank through a pipeline to be blended with the xylitol hydrogenation solution for reuse; wherein a purity of the crude xylitol crystals is within a range of 98.5-99.8%, and pH<5.0, and a purity of the primary centrifugal mother liquor is within a range of 88-92%; and
step 3, dissolving and recrystallizing the crude xylitol crystal, including delivering the crude xylitol crystal to a xylitol dissolution tank to be dissolved by added purified water, and then blending and mixing dissolved crude xylitol crystal with a secondary centrifugal mother liquor delivered from a second centrifuge in a certain proportion to obtain a dissolved xylitol mixture, the dissolved xylitol mixture being concentrated by a second evaporator, crystallized by a second crystallization kettle, centrifuged by a second centrifuge, dried by hot air of a hot air drying tank, and dried by cold air of a cold air fluidization bed in turn to obtain a high-quality xylitol crystal product, and the second centrifugal mother liquor obtained by the separation treatment of the second centrifuge back-setting to the xylitol dissolution tank through a pipeline to be mixed with a crude xylitol crystal solution for reuse; wherein a purity of the high-quality xylitol crystal product is greater than 99.8%, and pH>6.0.

Compared with the prior art, the preparation system and the preparation method for the high-quality xylitol crystals may evenly mix the hydrogenation solution with the primary centrifugal mother liquor to obtain the mixed material solution. The mixed material solution is refined and concentrated by the heat exchanger, the decolorization tank, the ion exchange system, the microporous filter, and the first evaporator to obtain the xylitol concentrate. The xylitol concentrate is crystalized in the first crystallization kettle according to a specific process to obtain the xylitol sugar paste. The xylitol sugar paste is separated by the first centrifuge to obtain the primary centrifugal mother liquor and the crude xylitol crystals. The primary centrifugal mother liquor is returned to the blending tank. The crude xylitol crystal is dried by the first fluidization bed and then dissolved in the xylitol dissolution tank by adding water, and evenly mixed with the second centrifugal mother liquor obtained by the second centrifuge to be fed into the second evaporator for concentration. The dissolved xylitol mixture is concentrated by the second evaporator and then the xylitol solution is recrystallized in the second crystallization kettle, and then separated by the second centrifuge to obtain the xylitol crystals and the second centrifugal mother liquor. The second centrifugal mother liquor is returned to the xylitol dissolution tank. The xylitol crystals are dried by the hot air drying tank and the cold air fluidization bed to obtain the high-quality xylitol crystal product. The purity of the prepared xylitol product in the present disclosure is further improved, and the pH of the xylitol crystals possesses better stability. After one month of standing, the pH of the xylitol crystals still maintains above 6.0. In addition, the sweetness and aftertaste of the xylitol product prepared by the preparation method are improved, while the dental erosion and numbness are reduced, and the flavor of the product is obviously improved.

### BRIEF DESCRIPTION OF THE DRAWINGS

FIG. 1 is a schematic diagram illustrating a principle of a preparation system for high-quality xylitol crystals; and
FIG. 2 is a schematic diagram illustrating flavor evaluation of xylitol crystals prepared in various Examples of the present disclosure and Comparative Examples.

### DETAILED DESCRIPTION

In order to more clearly illustrate the technical problems to be solved by the present disclosure, technical solutions, and beneficial effects, the present disclosure will be further illustrated in the description of the embodiments with reference to the accompanying drawings. It should be understood that the specific embodiments described herein are only intended to explain the present disclosure and are not intended to limit the present disclosure.

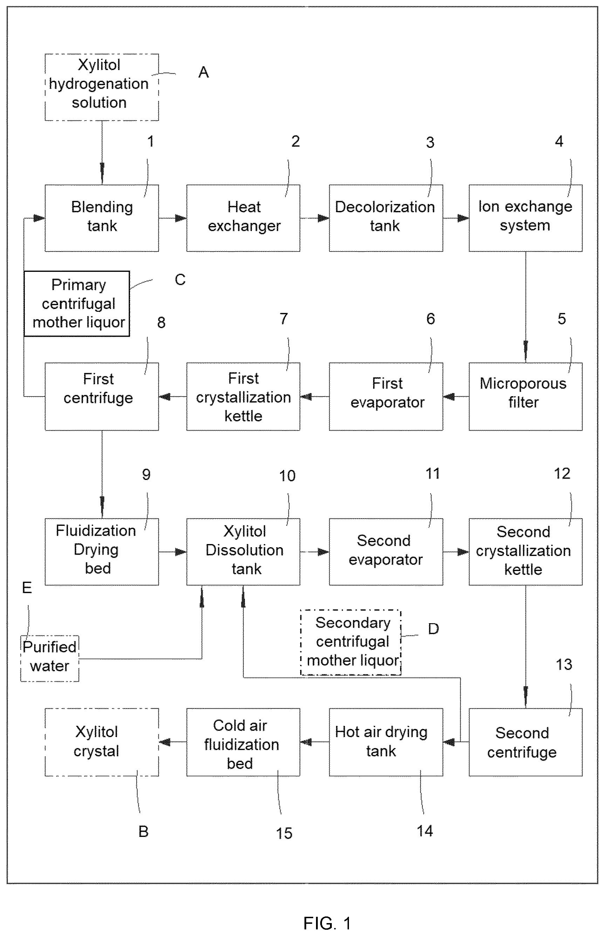

As illustrated in FIG. 1, a preferred embodiment of the present disclosure provides a preparation system for high-quality xylitol crystals. The preparation system may comprise a blending tank 1, a heat exchanger 2, a decolorization tank 3, an ion exchange system 4, a microporous filter 5, a first evaporator 6, a first crystallization kettle 7, a first centrifuge 8, a fluidization drying bed 9, a xylitol dissolution tank 10, a second evaporator 11, a second crystallization kettle 12, a second centrifuge 13, a hot air drying tank 14, and a cold air fluidization bed 15 which are sequentially connected through pipelines. The first centrifuge 8 and the second centrifuge 13 may be provided with a liquid outlet, respectively. The liquid outlet of the first centrifuge 8 may be connected with an inlet of the blending tank 1 through a pipeline. The liquid outlet of the second centrifuge 13 may be connected with an inlet of the xylitol dissolution tank 10 through a pipeline. The blending tank 1 may be provided with an inlet for a material to be treated. The xylitol dissolution tank 10 may be provided with a water inlet for purified water E. The cold air fluidization bed 15 may be provided with an outlet. The material to be treated may be a xylitol hydrogenation solution A. A material output from the outlet of the cold air fluidization bed 15 may be a xylitol crystal B.

A material output from the liquid outlet of the first centrifuge 8 may be a primary centrifugal mother liquor C, and a material output from the liquid outlet of the second centrifuge 13 may be a secondary centrifugal mother liquor D. The blending tank 1 may be configured to evenly mix the xylitol hydrogenation solution A with the primary centrifugal mother liquor C to obtain a mixed material solution.

The present disclosure further discloses a preparation method for high-quality xylitol crystals. The preparation method may comprise the following steps.
step 1, preparing a xylitol concentrate, including blending and mixing the xylitol hydrogenation solution A with the primary centrifugal mother liquor C delivered from the first centrifuge 8 in a certain proportion to obtain a mixed material solution, and then performing a heat exchange treatment by the heat exchanger 2, a decolorization treatment by the decolorization tank 3, an ion exchange treatment by the ion exchange system 4, a filtration treatment by the microporous filter 5, and an evaporation treatment by the first evaporator 6 on the mixed material solution, respectively, to obtain the xylitol concentrate. A concentration of the xylitol concentrate may be within a range of 1200-1400 g/L, and a purity of the xylitol concentrate may be within a range of 94-96%.
step 2, crystallizing the xylitol concentrate, including cooling and crystallizing the xylitol concentrate in the first crystallization kettle 7 for 8-12 h to obtain a xylitol sugar paste, performing a separation treatment by the first centrifuge 8 and a drying treatment by the fluidization drying bed 9 on the xylitol sugar paste to obtain crude xylitol crystals, and the primary centrifugal mother liquor obtained by the separation treatment of the first centrifuge 8 back-setting to the blending tank 1 through a pipeline to be blended with the xylitol hydrogenation solution A for reuse. A purity of the crude xylitol crystals may be within a range of 98.5-99.8%, and pH<5.0, and a purity of the primary centrifugal mother liquor may be within a range of 88-92%.
step 3, dissolving and recrystallizing the crude xylitol crystals, including delivering the crude xylitol crystals to the xylitol dissolution tank 10 to be dissolved by added purified water, and then blending and mixing dissolved crude xylitol crystals with the secondary centrifugal mother liquor D delivered from the second centrifuge 13 in a certain proportion to obtain a dissolved xylitol mixture, the dissolved xylitol mixture being concentrated by the second evaporator 11, crystallized by the second crystallization kettle 12, centrifuged by the second centrifuge 13, dried by hot air of the hot air drying tank 14, and dried by cold air of the cold air fluidization bed 15 in turn to obtain the high-quality xylitol crystal B, and the second centrifugal mother liquor D obtained by the separation treatment of the second centrifuge 13 back-setting to the xylitol dissolution tank 10 through a pipeline to be mixed with a crude xylitol crystal solution for reuse. A purity of the high-quality xylitol crystal B product may be >99.8%, and pH>6.0.

Specifically, in the step 1, a temperature of the xylitol concentrate may be within a range of 90-100 °C.

Specifically, in the step 2, xylitol crystal seeds may be added during a cooling and crystallization process in the first crystallization kettle 7, an addition proportion of the xylitol crystal seeds may be within a range of 0.001-0.002%, a mesh number of the xylitol crystal seeds may be within a range of 60-120, and a system temperature at a time of adding the xylitol crystal seeds may be within a range of 64-66 °C.

Specifically, in the step 3, the xylitol crystal seeds may be added during a crystallization process in the second crystallization kettle 12, an addition proportion of the xylitol crystal seeds may be within a range of 0.001-0.002%, a mesh number of the xylitol crystal seeds may be within a range of 60-120, and a system temperature at a time of adding the xylitol crystal seeds may be within a range of 64-66 °C.

The preparation system and the preparation method for the high-quality xylitol crystals are further illustrated through specific Examples below.

### Example 1

A first Example of a preparation method for high-quality xylitol crystals of the present disclosure comprises the following steps.

Step 11, a xylitol hydrogenation solution A was blended with a primary centrifugal mother liquor C in a certain proportion. A blending solution was refined and concentrated by the heat exchanger 2, the decolorization tank 3, the ion exchange system 4, the microporous filter 5, and the first evaporator 6 to obtain a xylitol concentrate with a temperature of 100 °C, a concentration of 1200 g/L, and a purity of 94%.

Step 12, a constant vacuum degree of the first crystallization kettle 7 was maintained at -0.095 MPa to further evaporate the xylitol concentrate. When a temperature of the first crystallization kettle 7 dropped to 65 °C, 0.001% xylitol crystal seeds (80-100 mesh) were added, and the system was maintained at a constant temperature of 65°C for evaporation and crystallization for 8 h. Finally, a crude xylitol crystal with a purity of 99.5% and a pH of 4.87 was obtained by a centrifugal separation treatment of the first centrifuge 8 and a fluidization drying treatment of the fluidization drying bed 9. At the same time, a primary centrifugal mother liquor C with a purity of 90% obtained by the centrifugal separation treatment of the first centrifuge 8 was backset to the blending tank 1.

Step 13, purified water was added to the crude xylitol crystal with the purity of 99.5% to be dissolved for blending with a secondary centrifugal mother liquor D to obtain a secondary blending solution with a xylitol content of 98.2%. The secondary blending solution was further concentrated by the second evaporator 11 to a concentration of 1350 g/L to enter the second crystallization kettle 12. The second crystallization kettle 12 was maintained at the constant vacuum degree of -0.095 MPa and the constant temperature of 65 °C, and 0.001% xylitol crystal seeds (80-100 mesh) were added for evaporation and crystallization for 8h. Finally, a xylitol crystal B with a purity of 99.97% was obtained by a centrifugal separation treatment of the second centrifuge 13, a hot air drying treatment of the second centrifugal drying tank 14, and a cold air drying treatment of the cold air fluidization bed 15. At the same time, the secondary centrifugal mother liquor D obtained by the centrifugal separation treatment of the second centrifuge 13 was backset to the xylitol dissolution tank 10.

An acidity of the prepared xylitol crystal B was measured according to the method in the Chinese Pharmacopoeia. A xylitol solution was prepared with freshly made ultrapure water in a proportion of 10 g crystal to 20 ml water. A pH of the xylitol solution in this Example was measured to be 6.06. After the obtained xylitol crystal B was placed at room temperature for one week, the pH of the xylitol solution was measured to be 6.03 using the same method. After the xylitol crystal was placed at room temperature for one month, the pH of the xylitol solution was measured to be 6.01 using the same method.

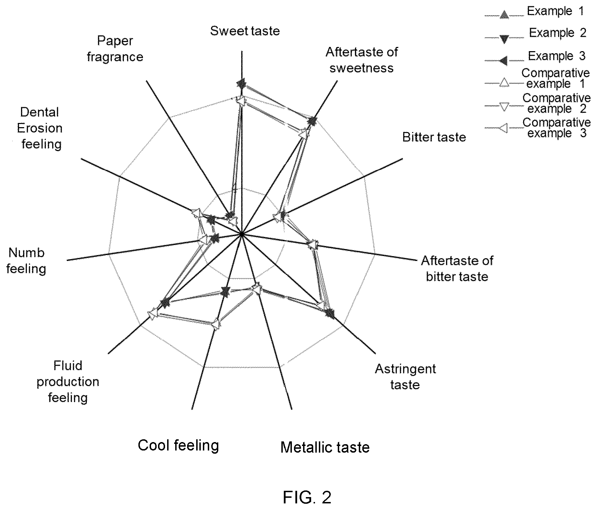

A flavor evaluation test was performed on the xylitol crystal prepared in Example 1, and results were obtained as shown in FIG. 2.

### Example 2

A second Example of a preparation method for high-quality xylitol crystals of the present disclosure comprises the following steps.

Step 21, a xylitol hydrogenation solution A was blended with a primary centrifugal mother liquor C in a certain proportion. A blending solution was refined and concentrated by the heat exchanger 2, the decolorization tank 3, the ion exchange system 4, the microporous filter 5, and the first evaporator 6 to obtain a xylitol concentrate with a temperature of 90 °C, a concentration of 1400 g/L, and a purity of 94%.

Step 22, a constant vacuum degree of the first crystallization kettle 7 was maintained at -0.095 MPa to further evaporate the xylitol concentrate. When a temperature of the first crystallization kettle 7 dropped to 64 °C, 0.001% xylitol crystal seeds (100-120 mesh) were added, and the system was maintained at a constant temperature of 64°C for evaporation and crystallization for 12 h. Finally, crude xylitol crystal with a purity of 99.6% and a pH of 4.9 were obtained by a centrifugal separation treatment of the first centrifuge 8 and a fluidization drying treatment of the fluidization drying bed 9. At the same time, a primary centrifugal mother liquor C with a purity of 89.5% obtained by the centrifugal separation treatment of the first centrifuge 8 was backset to the blending tank 1.

Step 23, purified water was added to the crude xylitol crystal with the purity of 99.6% to be dissolved for blending with a secondary centrifugal mother liquor D to obtain a secondary blending solution with a xylitol content of 98.4%. The secondary blending solution was further concentrated by the second evaporator 11 to a concentration of 1380 g/L to enter the second crystallization kettle 12. The second crystallization kettle 12 was maintained at the constant vacuum degree of -0.095 MPa and the constant temperature of 65 °C, and 0.001% xylitol crystal seeds (100-120 mesh) were added for evaporation and crystallization for 10h. Finally, a xylitol crystal B with a purity of 99.97% was obtained by a centrifugal separation treatment of the second centrifuge 13, a hot air drying treatment of the second centrifugal drying tank 14, and a cold air drying treatment of the cold air fluidization bed 15. At the same time, the secondary centrifugal mother liquor D obtained by the centrifugal separation treatment of the second centrifuge 13 was backset to the xylitol dissolution tank 10.

An acidity of prepared xylitol crystal B was measured according to the method in the Chinese Pharmacopoeia. A xylitol solution was prepared with freshly made ultrapure water in a proportion of 10 g crystal to 20 ml water. A pH of the xylitol solution in this Example was measured to be 6.12. After the obtained xylitol crystal B was placed at room temperature for one week, the pH of the xylitol solution was measured to be 6.08 using the same method. After the xylitol crystal was placed at room temperature for one month, the pH of the xylitol solution was measured to be 6.05 using the same method.

A flavor evaluation test was performed on the xylitol crystal prepared in Example 2, and results were obtained as shown in FIG. 2.

### Example 3

A third Example of a preparation method for high-quality xylitol crystals of the present disclosure comprises the following steps.

Step 31, a xylitol hydrogenation solution A was blended with a primary centrifugal mother liquor C in a certain proportion to obtain a blending solution with a xylitol content of 96%. The blending solution was refined and concentrated by the heat exchanger 2, the decolorization tank 3, the ion exchange system 4, the microporous filter 5, and the first evaporator 6 to obtain a xylitol concentrate with a temperature of 95 °C and a concentration of 1350 g/L.

Step 32, a constant vacuum degree of the first crystallization kettle 7 was maintained at -0.095 MPa to further evaporate the xylitol concentrate. When a temperature of the first crystallization kettle 7 dropped to 66 °C, 0.002% xylitol crystal seeds (60-80 mesh) were added, and the system was maintained at a constant temperature of 66°C for evaporation and crystallization for 10 h. Finally, crude xylitol crystal with a purity of 99.7% and a pH of 4.99 was obtained by a centrifugal separation treatment of the first centrifuge 8 and a fluidization drying treatment of the fluidization drying bed 9. At the same time, a primary centrifugal mother liquor C with a purity of 92% obtained by the centrifugal separation treatment of the first centrifuge 8 was backset to the blending tank 1.

Step 33, purified water was added to the crude xylitol crystal with the purity of 99.7% to be dissolved for blending with a secondary centrifugal mother liquor D to obtain a secondary blending solution with a xylitol content of 98.4%. The secondary blending solution was further concentrated by the second evaporator 11 to a concentration of 1400 g/L to enter the second crystallization kettle 12. The second crystallization kettle 12 was maintained at the constant vacuum degree of -0.095 MPa and the constant temperature of 65 °C, and 0.002% xylitol crystal seeds (60-80 mesh) were added for evaporation and crystallization for 10h. Finally, a xylitol crystal B with a purity of 100% was obtained by a centrifugal separation treatment of the second centrifuge 13, a hot air drying treatment of the second centrifugal drying tank 14, and a cold air drying treatment of the cold air fluidization bed 15. At the same time, the secondary centrifugal mother liquor D obtained by the centrifugal separation treatment of the second centrifuge 13 was backset to the xylitol dissolution tank 10.

An acidity of prepared xylitol crystal B was measured according to the method in the Chinese Pharmacopoeia. A xylitol solution was prepared with freshly made ultrapure water in a proportion of 10 g crystal to 20 ml water. A pH of the xylitol solution in this Example was measured to be 6.09. After the obtained xylitol crystal B was placed at room temperature for one week, the pH of the xylitol solution was measured to be 6.07 using the same method. After the xylitol crystal was placed at room temperature for one month, the pH of the xylitol solution was measured to be 6.05 using the same method.

A flavor evaluation test was performed on the xylitol crystal prepared in Example 3, and results were obtained as shown in FIG. 2.

The following comparative experiments were conducted to further illustrate the improvement effects of the present disclosure.

### Comparative Example 1

A first comparative example of a preparation method for xylitol crystals of the present disclosure comprises the following steps.

Step D11, a xylitol hydrogenation solution A was blended with a primary centrifugal mother liquor C in a certain proportion to obtain a blending solution with a xylitol content of 94%. A blending solution was refined and concentrated by the heat exchanger 2, the decolorization tank 3, the ion exchange system 4, the microporous filter 5, and the first evaporator 6 to obtain a xylitol concentrate with a temperature of 95 °C and a concentration of 1300 g/L.

Step D12, a constant vacuum degree of the first crystallization kettle 7 was maintained at -0.095 MPa to further evaporate the xylitol concentrate. When a temperature of the first crystallization kettle 7 dropped to 64 °C, 0.001% xylitol crystal seeds (80-100 mesh) were added, and the system was maintained at a constant temperature of 64°C for evaporation and crystallization for 10 h. Finally, xylitol crystal with a purity of 99.8% was obtained by a centrifugal separation treatment of the first centrifuge 8 and a fluidization drying treatment of the fluidization drying bed 9. At the same time, a primary centrifugal mother liquor C with a purity of 90% obtained by the centrifugal separation treatment of the first centrifuge 8 was backset to the blending tank 1.

An acidity of prepared xylitol crystal was measured according to the method in the Chinese Pharmacopoeia. A xylitol solution was prepared with freshly made ultrapure water in a proportion of 10 g crystal to 20 ml water. A pH of the xylitol solution in this comparative example was measured to be 5.02. After the obtained xylitol crystal was placed at room temperature for one week, the pH of the xylitol solution was measured to be 4.85 using the same method. After the xylitol crystal were placed at room temperature for one month, the pH of the xylitol solution was measured to be 4.64 using the same method.

A flavor evaluation test was performed on the xylitol crystal prepared in comparative example 1, and results were obtained as shown in FIG. 2.

### Comparative Example 2

A second comparative example of a preparation method for xylitol crystals of the present disclosure comprises the following steps.

Step D21, a xylitol hydrogenation solution A was blended with a primary centrifugal mother liquor C in a certain proportion to obtain a blending solution with a xylitol content of 94.5%. A blending solution was refined and concentrated by the heat exchanger 2, the decolorization tank 3, the ion exchange system 4, the microporous filter 5, and the first evaporator 6 to obtain a xylitol concentrate with a temperature of 100 °C and a concentration of 1200 g/L.

Step D22, a constant vacuum degree of the first crystallization kettle 7 was maintained at -0.095 MPa to further evaporate the xylitol concentrate. When a temperature of the first crystallization kettle 7 dropped to 65 °C, 0.002% xylitol crystal seeds (100-120 mesh) were added, and the system was maintained at a constant temperature of 65°C for evaporation and crystallization for 8 h. Finally, xylitol crystal with a purity of 99.75% was obtained by a centrifugal separation treatment of the first centrifuge 8 and a fluidization drying treatment of the fluidization drying bed 9. At the same time, a primary centrifugal mother liquor C with a purity of 91% obtained by the centrifugal separation treatment of the first centrifuge 8 was backset to the blending tank 1.

An acidity of prepared xylitol crystal was measured according to the method in the Chinese Pharmacopoeia. A xylitol solution was prepared with freshly made ultrapure water in a proportion of 10 g crystal to 20 ml water. A pH of the xylitol solution in this comparative example was measured to be 5.04. After the obtained xylitol crystal was placed at room temperature for one week, the pH of the xylitol solution was measured to be 4.81 using the same method. After the xylitol crystal was placed at room temperature for one month, the pH of the xylitol solution was measured to be 4.68 using the same method.

A flavor evaluation test was performed on the xylitol crystal prepared in comparative example 2, and results were obtained as shown in FIG. 2.

### Comparative Example 3

A third comparative example of a preparation method for xylitol crystals of the present disclosure comprises the following steps.

Step D31, a xylitol hydrogenation solution A was blended with a primary centrifugal mother liquor C in a certain proportion to obtain a blending solution with a xylitol content of 96%. A blending solution was refined and concentrated by the heat exchanger 2, the decolorization tank 3, the ion exchange system 4, the microporous filter 5, and the first evaporator 6 to obtain a xylitol concentrate with a temperature of 92 °C and a concentration of 1400 g/L.

Step D32, a constant vacuum degree of the first crystallization kettle 7 was maintained at -0.095 MPa to further evaporate the xylitol concentrate. When a temperature of the first crystallization kettle 7 dropped to 66 °C, 0.001% xylitol crystal seeds (100-120 mesh) were added, and the system was maintained at a constant temperature of 66°C for evaporation and crystallization for 12 h. Finally, xylitol crystal with a purity of 99.7% was obtained by a centrifugal separation treatment of the first centrifuge 8 and a fluidization drying treatment of the fluidization drying bed 9. At the same time, a primary centrifugal mother liquor C with a purity of 92% obtained by the centrifugal separation treatment of the first centrifuge 8 was backset to the blending tank 1.

An acidity of prepared xylitol crystal was measured according to the method in the Chinese Pharmacopoeia. A xylitol solution was prepared with freshly made ultrapure water in a proportion of 10 g crystals to 20 ml water. A pH of the xylitol solution in this comparative example was measured to be 5.03. After the obtained xylitol crystal was placed at room temperature for one week, the pH of the xylitol solution was measured to be 4.90 using the same method. After the xylitol crystal was placed at room temperature for one month, the pH of the xylitol solution was measured to be 4.73 using the same method.

A flavor evaluation test was performed on the xylitol crystal prepared in comparative example 3, and results were obtained as shown in FIG. 2.

The results of pH and flavor evaluation of the xylitol crystals prepared in the above examples and comparative examples are summarized as shown in Table 3 and FIG. 2. It can be seen from Table 3 that the pH of the xylitol crystals of Examples 1-3 are above 6.0, and the pH of the xylitol crystals decrease by no more than 0.1 after one month of placement; while the pH of the xylitol crystals of comparative examples 1-3 are only about 5.0, and the pH of the xylitol crystals decrease by more than 0.3 after one month of placement. These data indicates that the pH of the xylitol crystals can be significantly increased and stabilized by recrystallization. In addition, it can be seen from the flavor evaluation radar chart in FIG. 2 that sweetness, aftertaste and other attributes of the xylitol crystals prepared in Examples 1-3 that are preferred by consumers are enhanced compared to those of comparative examples, and unfavorable attributes such as dental erosion, numbness, or the like were weakened compared to those of comparative examples, which further indicates that the xylitol crystals prepared by the method described in the present disclosure have significantly improved flavor.

**Table 3 Results of pH of the xylitol crystals prepared in the above examples and comparative examples**

| Implementation sample of xylitol solution | pH of xylitol of the day | pH of xylitol after one week | pH of xylitol after one month |
|---|---|---|---|
| Example 1 | 6.06 | 6.03 | 6.01 |
| Example 2 | 6.12 | 6.08 | 6.05 |
| Example 3 | 6.09 | 6.07 | 6.05 |
| Comparative example 1 | 5.02 | 4.85 | 4.64 |
| Comparative example 2 | 5.04 | 4.81 | 4.68 |
| Comparative example 3 | 5.03 | 4.90 | 4.73 |

The above descriptions are only preferred embodiments of the present disclosure and are not intended to limit the present disclosure.

## Claims

1. A preparation system for a high-quality xylitol crystal, comprising: a blending tank, a heat exchanger, a decolorization tank, an ion exchange system, a microporous filter, a first evaporator, a first crystallization kettle, a first centrifuge, a fluidization drying bed, a xylitol dissolution tank, a second evaporator, a second crystallization kettle, a second centrifuge, a hot air drying tank, and a cold air fluidization bed which are sequentially connected through pipelines, wherein the first centrifuge and the second centrifuge are provided with a liquid outlet, respectively, the liquid outlet of the first centrifuge is connected with an inlet of the blending tank through a pipeline, the liquid outlet of the second centrifuge is connected with an inlet of the xylitol dissolution tank through a pipeline, the blending tank is provided with an inlet for a material to be treated, the xylitol dissolution tank is provided with a water inlet for purified water, the cold air fluidization bed is provided with an outlet, the material to be treated is a xylitol hydrogenation solution, and a material output from the outlet of the cold air fluidization bed is a xylitol crystal product.

2. A preparation method for a high-quality xylitol crystal, comprising:
step 1, preparing a xylitol concentrate, including blending and mixing a xylitol hydrogenation solution with a primary centrifugal mother liquor delivered from a first centrifuge in a certain proportion to obtain a mixed material solution, and then performing a heat exchange treatment by a heat exchanger, a decolorization treatment by a decolorization tank, an ion exchange treatment by an ion exchange system, a filtration treatment by a microporous filter, and an evaporation treatment by a first evaporator on the mixed material solution, respectively, to obtain the xylitol concentrate; wherein a concentration of the xylitol concentrate is within a range of 1200-1400 g/L, and a purity of the xylitol concentrate is within a range of 94-96%;
step 2, crystallizing the xylitol concentrate, including cooling and crystallizing the xylitol concentrate in a first crystallization kettle for 8-12 h to obtain a xylitol sugar paste, performing a separation treatment by the first centrifuge and a drying treatment by a fluidization drying bed on the xylitol sugar paste to obtain a crude xylitol crystal, and the primary centrifugal mother liquor obtained by the separation treatment of the first centrifuge back-setting to the blending tank through a pipeline to be blended with the xylitol hydrogenation solution for reuse; wherein a purity of the crude xylitol crystal is within a range of 98.5-99.8%, and pH<5.0, and a purity of the primary centrifugal mother liquor is within a range of 88-92%; and
step 3, dissolving and recrystallizing the crude xylitol crystal, including delivering the crude xylitol crystal to a xylitol dissolution tank to be dissolved by added purified water to obtain a crude xylitol solution, and then blending and mixing the crude xylitol solution with a secondary centrifugal mother liquor delivered from a second centrifuge in a certain proportion to obtain a dissolved xylitol mixture, the dissolved xylitol mixture being concentrated by a second evaporator, crystallized by a second crystallization kettle, centrifuged by a second centrifuge, dried by hot air of a hot air drying tank, and dried by cold air of a cold air fluidization bed in turn to obtain a high-quality xylitol crystal product, and the second centrifugal mother liquor obtained by the separation treatment of the second centrifuge back-setting to the xylitol dissolution tank through a pipeline to be mixed with the crude xylitol solution for reuse; wherein a purity of the high-quality xylitol crystal product is greater than 99.8%, and pH>6.0.

3. The preparation method of claim 2, wherein in the step 1, a temperature of the xylitol concentrate is within a range of 90-100 °C.

4. The preparation method of claim 2, wherein in the step 2, xylitol crystal seeds are added during a cooling and crystallization process, an addition proportion of the xylitol crystal seeds is within a range of 0.001-0.002%, a mesh number of the xylitol crystal seeds is within a range of 60-120 mesh, and a system temperature at a time of adding the xylitol crystal seeds is within a range of 64-66°C.

5. The preparation method of claim 1, wherein in the step 3, xylitol crystal seeds are added during a crystallization process in the second crystallization kettle, an addition proportion of the xylitol crystal seeds is within a range of 0.001-0.002%, a mesh number of the xylitol crystal seeds is within a range of 60-120 mesh, and a system temperature at a time of adding the xylitol crystal seeds is within a range of 64-66 °C.

## Patentansprüche

1. Ein Herstellungssystem für einen hochwertigen Xylitkristall, umfassend: einen Mischbehälter, einen Wärmetauscher, einen Entfärbungsbehälter, ein Ionenaustauschsystem, einen mikroporösen Filter, einen ersten Verdampfer, einen ersten Kristallisationskessel, eine erste Zentrifuge, ein Wirbelschichttrocknungsbett, einen Xylit-Auflösebehälter, einen zweiten Verdampfer, einen zweiten Kristallisationskessel, eine zweite Zentrifuge, einen Heißlufttrocknungsbehälter und ein Kaltluft-Wirbelschichtbett, die der Reihe nach über Rohrleitungen miteinander verbunden sind, wobei die erste Zentrifuge und die zweite Zentrifuge jeweils mit einem Flüssigkeitsauslass versehen sind, der Flüssigkeitsauslass der ersten Zentrifuge über eine Rohrleitung mit einem Einlass des Mischbehälters verbunden ist, der Flüssigkeitsauslass der zweiten Zentrifuge über eine Rohrleitung mit einem Einlass des Xylit-Auflösebehälters verbunden ist, der Mischbehälter mit einem Einlass für ein zu behandelndes Material versehen ist, der Xylit-Auflösebehälter mit einem Wassereinlass für gereinigtes Wasser versehen ist, das Kaltluft-Wirbelschichtbett mit einem Auslass versehen ist, das zu behandelnde Material eine Xylit-Hydrierungslösung ist, und ein aus dem Auslass des Kaltluft-Wirbelschichtbetts ausgegebenes Material ein Xylitkristallprodukt ist.

2. Ein Herstellungsverfahren für einen hochwertigen Xylitkristall, umfassend:
Schritt 1, Herstellen eines Xylitkonzentrats, umfassend das Vermischen und Mischen einer Xylit-Hydrierungslösung mit einer von einer ersten Zentrifuge geförderten primären Zentrifugalmutterlauge in einem bestimmten Verhältnis, um eine gemischte Materiallösung zu erhalten, und anschließend jeweils Durchführen einer Wärmeaustauschbehandlung mittels eines Wärmetauschers, einer Entfärbungsbehandlung mittels eines Entfärbungsbehälters, einer lonenaustauschbehandlung mittels eines Ionenaustauschsystems, einer Filtrationsbehandlung mittels eines mikroporösen Filters und einer Verdampfungsbehandlung mittels eines ersten Verdampfers an der gemischten Materiallösung, um das Xylitkonzentrat zu erhalten; wobei eine Konzentration des Xylitkonzentrats in einem Bereich von 1200-1400 g/L liegt, und eine Reinheit des Xylitkonzentrats in einem Bereich von 94-96 % liegt;
Schritt 2, Kristallisieren des Xylitkonzentrats, umfassend das Kühlen und Kristallisieren des Xylitkonzentrats in einem ersten Kristallisationskessel für 8-12 h, um eine Xylit-Zuckerpaste zu erhalten, Durchführen einer Trennbehandlung mittels der ersten Zentrifuge und einer Trocknungsbehandlung mittels eines Wirbelschichttrocknungsbetts an der Xylit-Zuckerpaste, um einen Roh-Xylitkristall zu erhalten, und die primäre Zentrifugalmutterlauge, die durch die Trennbehandlung der ersten Zentrifuge erhalten wird, über eine Rohrleitung in den Mischbehälter zurückzuführen, um mit der Xylit-Hydrierungslösung zum Wiederverwenden gemischt zu werden; wobei eine Reinheit des Roh-Xylitkristalls in einem Bereich von 98,5-99,8% liegt, und pH<5,0, und eine Reinheit der primären Zentrifugalmutterlauge in einem Bereich von 88-92 % liegt; und
Schritt 3, Lösen und Rekristallisieren des Roh-Xylitkristalls, umfassend das Fördern des Roh-Xylitkristalls zu einem Xylit-Auflösebehälter, um durch zugesetztes gereinigtes Wasser gelöst zu werden und eine Roh-Xylitlösung zu erhalten, und anschließend das Vermischen und Mischen der Roh-Xylitlösung mit einer von einer zweiten Zentrifuge geförderten sekundären Zentrifugalmutterlauge in einem bestimmten Verhältnis, um eine gelöste Xylitmischung zu erhalten, wobei die gelöste Xylitmischung der Reihe nach mittels eines zweiten Verdampfers konzentriert, mittels eines zweiten Kristallisationskessels kristallisiert, mittels einer zweiten Zentrifuge zentrifugiert, mittels heißer Luft eines Heißlufttrocknungsbehälters getrocknet und mittels kalter Luft eines Kaltluft-Wirbelschichtbetts getrocknet wird, um ein hochwertiges Xylitkristallprodukt zu erhalten, und die sekundäre Zentrifugalmutterlauge, die durch die Trennbehandlung der zweiten Zentrifuge erhalten wird, über eine Rohrleitung in den Xylit-Auflösebehälter zurückgeführt wird, um mit der Roh-Xylitlösung zum Wiederverwenden gemischt zu werden; wobei eine Reinheit des hochwertigen Xylitkristallprodukts größer als 99,8 % ist, und pH>6,0.

3. Das Herstellungsverfahren nach Anspruch 2, wobei in Schritt 1 eine Temperatur des Xylitkonzentrats in einem Bereich von 90-100 °C liegt.

4. Das Herstellungsverfahren nach Anspruch 2, wobei in Schritt 2 während eines Kühl- und Kristallisationsprozesses Xylitkristallsaaten zugesetzt werden, ein Zugabeverhältnis der Xylitkristallsaaten in einem Bereich von 0,001-0,002 % liegt, eine Maschenzahl der Xylitkristallsaaten in einem Bereich von 60-120 mesh liegt, und eine Systemtemperatur zum Zeitpunkt des Zusetzens der Xylitkristallsaaten in einem Bereich von 64-66 °C liegt.

5. Das Herstellungsverfahren nach Anspruch 1, wobei in Schritt 3 während eines Kristallisationsprozesses in dem zweiten Kristallisationskessel Xylitkristallsaaten zugesetzt werden, ein Zugabeverhältnis der Xylitkristallsaaten in einem Bereich von 0,001-0,002 % liegt, eine Maschenzahl der Xylitkristallsaaten in einem Bereich von 60-120 mesh liegt, und eine Systemtemperatur zum Zeitpunkt des Zusetzens der Xylitkristallsaaten in einem Bereich von 64-66 °C liegt.

## Revendications

1. Un système de préparation d'un cristal de xylitol de haute qualité, comprenant : une cuve de mélange, un échangeur de chaleur, une cuve de décoloration, un système d'échange ionique, un filtre microporeux, un premier évaporateur, une première cuve de cristallisation, une première centrifugeuse, un lit fluidisé de séchage, une cuve de dissolution de xylitol, un deuxième évaporateur, une deuxième cuve de cristallisation, une deuxième centrifugeuse, une cuve de séchage à air chaud, et un lit fluidisé à air froid qui sont reliés séquentiellement par des conduites, dans lequel la première centrifugeuse et la deuxième centrifugeuse sont respectivement pourvues d'une sortie de liquide, la sortie de liquide de la première centrifugeuse est reliée à une entrée de la cuve de mélange par une conduite, la sortie de liquide de la deuxième centrifugeuse est reliée à une entrée de la cuve de dissolution de xylitol par une conduite, la cuve de mélange est pourvue d'une entrée pour un matériau à traiter, la cuve de dissolution de xylitol est pourvue d'une entrée d'eau pour de l'eau purifiée, le lit fluidisé à air froid est pourvu d'une sortie, le matériau à traiter est une solution d'hydrogénation de xylitol, et un matériau délivré par la sortie du lit fluidisé à air froid est un produit cristallin de xylitol.

2. Un procédé de préparation d'un cristal de xylitol de haute qualité, comprenant :
Étape 1, préparer un concentré de xylitol, comprenant le mélange et la mise en mélange d'une solution d'hydrogénation de xylitol avec une liqueur mère centrifuge primaire acheminée depuis une première centrifugeuse dans une certaine proportion pour obtenir une solution de matière mélangée, puis effectuer respectivement un traitement d'échange thermique par un échangeur de chaleur, un traitement de décoloration par une cuve de décoloration, un traitement d'échange ionique par un système d'échange ionique, un traitement de filtration par un filtre microporeux, et un traitement d'évaporation par un premier évaporateur sur la solution de matière mélangée, afin d'obtenir le concentré de xylitol ; dans lequel une concentration du concentré de xylitol est dans une plage de 1200-1400 g/L, et une pureté du concentré de xylitol est dans une plage de 94-96 % ;
Étape 2, cristalliser le concentré de xylitol, comprenant le refroidissement et la cristallisation du concentré de xylitol dans une première cuve de cristallisation pendant 8-12 h afin d'obtenir une pâte sucrée de xylitol, l'exécution d'un traitement de séparation par la première centrifugeuse et d'un traitement de séchage par un lit fluidisé de séchage sur la pâte sucrée de xylitol afin d'obtenir un cristal brut de xylitol, et la liqueur mère centrifuge primaire obtenue par le traitement de séparation de la première centrifugeuse étant renvoyée vers la cuve de mélange par une conduite pour être mélangée avec la solution d'hydrogénation de xylitol en vue d'une réutilisation ; dans lequel une pureté du cristal brut de xylitol est dans une plage de 98,5-99,8 %, et pH<5,0, et une pureté de la liqueur mère centrifuge primaire est dans une plage de 88-92 % ; et
Étape 3, dissoudre et recristalliser le cristal brut de xylitol, comprenant l'acheminement du cristal brut de xylitol vers une cuve de dissolution de xylitol pour être dissous par de l'eau purifiée ajoutée afin d'obtenir une solution brute de xylitol, puis le mélange et la mise en mélange de la solution brute de xylitol avec une liqueur mère centrifuge secondaire acheminée depuis une deuxième centrifugeuse dans une certaine proportion afin d'obtenir un mélange dissous de xylitol, le mélange dissous de xylitol étant concentré par un deuxième évaporateur, cristallisé par une deuxième cuve de cristallisation, centrifugé par une deuxième centrifugeuse, séché par air chaud d'une cuve de séchage à air chaud, et séché par air froid d'un lit fluidisé à air froid, successivement, afin d'obtenir un produit cristallin de xylitol de haute qualité, et la liqueur mère centrifuge secondaire obtenue par le traitement de séparation de la deuxième centrifugeuse étant renvoyée vers la cuve de dissolution de xylitol par une conduite pour être mélangée avec la solution brute de xylitol en vue d'une réutilisation ; dans lequel une pureté du produit cristallin de xylitol de haute qualité est supérieure à 99,8 %, et pH>6,0.

3. Le procédé de préparation selon la revendication 2, dans lequel, à l'Étape 1, une température du concentré de xylitol est dans une plage de 90-100 °C.

4. Le procédé de préparation selon la revendication 2, dans lequel, à l'Étape 2, des germes cristallins de xylitol sont ajoutés pendant un processus de refroidissement et de cristallisation, une proportion d'ajout des germes cristallins de xylitol est dans une plage de 0,001-0,002 %, un nombre de mailles des germes cristallins de xylitol est dans une plage de 60-120 mesh, et une température du système au moment de l'ajout des germes cristallins de xylitol est dans une plage de 64-66 °C.

5. Le procédé de préparation selon la revendication 1, dans lequel, à l'Étape 3, des germes cristallins de xylitol sont ajoutés pendant un processus de cristallisation dans la deuxième cuve de cristallisation, une proportion d'ajout des germes cristallins de xylitol est dans une plage de 0,001-0,002 %, un nombre de mailles des germes cristallins de xylitol est dans une plage de 60-120 mesh, et une température du système au moment de l'ajout des germes cristallins de xylitol est dans une plage de 64-66 °C.
